# EUROPEAN PATENT SPECIFICATION

(11) **EP 2 217 293 B1**
(45) Date of publication and mention of the grant of the patent: **22.05.2019**
(21) Application number: 08863954.7
(22) Date of filing: 10.11.2008
(51) Int. Cl.: A61L 27/24, A61L 27/36

(54) **BONE MATRIX COMPOSITIONS HAVING NANOSCALE TEXTURED SURFACES**
KNOCHENMATRIXZUSAMMENSETZUNGEN MIT TEXTURIERTEN NANOSKALIGEN OBERFLÄCHEN
COMPOSITIONS DE MATRICE OSSEUSE AYANT DES SURFACES TEXTURÉES À L'ÉCHELLE NANOMÉTRIQUE

(30) Priority: 09.11.2007 US 986839 P; 16.06.2008 US 140062
(43) Date of publication of application: 18.08.2010
(73) Proprietor: Warsaw Orthopedic, Inc., Warsaw, IN 46581 (US)
(72) Inventor: WEI, Guobao, Eatontown NJ 07724 (US); BEHNAM, Keyvan, Red Bank NJ 07701 (US); FORSYTH, Nanette, Bayville NY 08721 (US)
(74) Representative: FRKelly
(86) International application number: PCT/US2008/083005
(87) International publication number: WO 2009/082554

(56) References cited:
- WO-A1-03/025271
- WO-A2-01/79342
- WO-A2-2005/081699
- US-A- 4 394 370
- US-A- 5 902 562
- US-A1- 2007 125 700

## Description

### FIELD OF THE INVENTION

The present invention relates generally to methods for the production of demineralized bone matrix compositions.

### BACKGROUND

### Overview of Bone Grafts

The rapid and effective repair of bone defects caused by injury, disease, wounds, or surgery is a goal of orthopedic surgery. Toward this end, a number of compositions and materials have been used or proposed for use in the repair of bone defects. The biological, physical, and mechanical properties of the compositions and materials are among the major factors influencing their suitability and performance in various orthopedic applications.

Autologous cancellous bone ("ACB"), also known as autograft or autogenous bone, is considered the gold standard for bone grafts. ACB is osteoinductive and nonimmunogenic, and, by definition, has all of the appropriate structural and functional characteristics appropriate for the particular recipient. Unfortunately, ACB is only available in a limited number of circumstances. Some individuals lack ACB of appropriate dimensions and quality for transplantation, and donor site pain and morbidity can pose serious problems for patients and their physicians.

Much effort has been invested in the identification and development of alternative bone graft materials. Urist published seminal articles on the theory of bone induction and a method for decalcifying bone, i.e., making demineralized bone matrix (DBM). Urist M.R., Bone Formation by Autoinduction, Science 1965; 150(698):893-9; Urist M.R. et al., The Bone Induction Principle, Clin. Orthop. Rel. Res. 53:243-283, 1967. DBM is an osteoinductive material in that it induces bone growth when implanted in an ectopic site of a rodent, at least partially because of the osteoinductive factors contained within the DBM. Honsawek et al. (2000). It is now known that there are numerous osteoinductive factors, e.g., BMP2, BMP4, BMP6, BMP7, which are part of the transforming growth factor-beta (TGF-beta) superfamily. BMP-2 has become the most important and widely studied of the BMP family of proteins. There are also other proteins present in DBM that are not osteoinductive alone but still contribute to bone growth, including fibroblast growth factor-2 (FGF-2), insulin-like growth factor-I and -II (IGF-I and IGF-II), platelet derived growth factor (PDGF), and transforming growth factor-beta 1 (TGF-beta.1).

Accordingly, a known technique for promoting the process of incorporation of osteoimplants is demineralization over outer surfaces, inner surfaces, or the entire volume of the implant. Various methods for demineralizing bone have been discussed. In this regard see, Lewandrowski et al., J. Biomed Materials Res, 31, pp. 365 372 (1996); Lewandrowski et al., Calcified Tiss. Int., 61, pp. 294 297 (1997); Lewandrowski et al., J. Ortho. Res., 15, pp. 748 756 (1997).

DBM implants have been reported to be particularly useful (see, for example, U.S. Patent Nos. 4,394,370, 4,440,750, 4,485,097, 4,678,470, and 4,743,259; Mulliken et al., Calcif Tissue Int. 33:71, 1981; Neigel et al., Opthal. Plast. Reconstr. Surg. 12:108, 1996; Whiteman et al., J. Hand. Surg. 18B:487, 1993; Xiaobo et al., Clin. Orthop. 293:360, 1993). DBM typically is derived from cadavers. The bone is removed aseptically and treated to kill any infectious agents. The bone is particulated by milling or grinding, and then the mineral component is extracted by various methods, such as by soaking the bone in an acidic solution. The remaining matrix is malleable and can be further processed and/or formed and shaped for implantation into a particular site in the recipient. The demineralized bone particles or fibers can be formulated with biocompatible excipients to enhance surgical handling properties and conformability to the defect or surgery site. Demineralized bone prepared in this manner contains a variety of components including proteins, glycoproteins, growth factors, and proteoglycans. Following implantation, the presence of DBM induces cellular recruitment to the site of injury. The recruited cells may eventually differentiate into bone forming cells. Such recruitment of cells leads to an increase in the rate of wound healing and, therefore, to faster recovery for the patient.

WO0179342 concerns a method for preparing cross-linked collagen, and cross-linked collagen products. The method includes incubating collagen in a solution including water, at least one polar solvent and at least one sugar, to form cross-linked collagen. The solution may include a buffer having a suitable pH and ionic strength. The method may include removing excess unreacted sugar(s) and polar solvent(s) by washing the cross-linked collagen or by other methods. The method may also include dehydrating the cross-linked collagen, and may include subjecting the cross-linked collagen to critical point drying, or subjecting the collagen to drying or freeze-drying prior to cross-linking. The collagen may be prepared from atelopeptide collagen to reduce antigenicity, but may also be prepared from other suitable collagen types. The concentration and type of the polar solvent(s), the concentration and type of the reducing sugar(s), and the incubation duration may be varied to control the degree of cross-linking. The cross-linked collagen product may be in the form of a wet or dry matrix or membrane or may be suspended in a liquid in the form of an injectable preparation. The method may be applied to collagenous proteins and collagen-like peptides.

WO2005081699 concerns a composite comprising an organic fluid-swellable, fibrous matrix, such as collagen, and a mineral phase, such as calcium carbonate or phosphate mineral phase, for use as a biomimetic of bone. In another aspect, the subject invention concerns a process for making a composite involving the inclusion of acidic polymers to a supersaturated mineralizing solution, in order to induce an amorphous liquid-phase precursor to the inorganic mineral, which is then absorbed (pulled by capillary action) into the organic matrix. Advantageously, once solidified, a high mineral content can be achieved, with the inorganic mineral crystals embedded within the collagen fibers (intrafibrillarly) and oriented such that they are aligned along the long axes of the fibers of the organic matrix, thereby closely mimicking the natural structure of bone. WO2005081699 further concerns a method of treating a patient suffering from a bone defect by applying a biomimetic composite to the bone defect site.

US2007125700 concerns novel composite materials comprising a porous support and a nanoweb coating and/or interpenetrating the porous support. The nanoweb is comprised of fibrous structures derived from gelation and drying of supramolecular assemblies of non-covalently bonded organogelators. Typical organogelators useful in the invention include those that assemble via hydrogen bonding and pi-stacking. Methods for preparing the composite materials are also disclosed that include critical point drying of the gelled nanowebs with carbon dioxide. The composites are useful as filters for gaseous and liquid fluids, as barrier fabrics, and as cleaning wipes.

U.S. Patent Nos. 4,394,370 concerns complexes of reconstituted collagen and demineralized bone particles or reconstituted collagen and a solubilized bone morphogenetic protein fabricated in a sponge suitable for in vivo implantation in osseous defects. Both demineralized bone particles (DBP) and bone morphogenetic protein have demonstrated the ability to induce the formation of osseous tissue in animal and human experiments. Reconstituted collagen conjugate is highly biocapatible and can be fabricated in a variety of configurations, especially as a sponge. This material can be used as a grafting implant in plastic and reconstructive surgery, periodontal bone grafting, and in endodontic procedures. Structural durability is enhanced by cross-linking with glutaraldehyde which is also used to sterilize and disinfect the collagen conjugate prior to implantation.

### BRIEF SUMMARY

Bone matrix compositions and, more specifically, bone matrix compositions having textured surfaces attractive to cells are disclosed and methods for their production are provided.

The invention provides, in a first aspect, a method for producing a demineralized bone matrix composition, the method comprising: providing bone; demineralizing the bone; drying the bone such that, after drying, the bone matrix has a residual moisture content of 15% or less; and imparting nanostructures to the bone to provide a nanoscale textured surface; wherein the imparting step is selected from adsorption, coating, precipitating; treating biomacromolecules of the bone to form surfaces with charged polycationic or anionic polyelectrolytes or with different hydrophilic and hydrophobic domains so that biomacromolecules with opposite charges or hydrophilic / hydrophobic properties are immobilized on the bone surface, and electrospinning. The invention also provides, in a second aspect, a demineralized bone matrix composition produced according to the method of the first aspect of the invention.

In some embodiments, nanostructures are imparted to bone matrix compositions wherein the nanofibrous properties on the surface may be compromised. In other embodiments, the bone is processed in a manner so as to expose and/or maintain the nanofibrous structure of the bone. Generally, these methods may be applied to demineralized bone including partially or surface demineralized bone. Further, these methods may be applied to particulated or monolithic bone.

A demineralized bone matrix composition comprising demineralized bone particles exhibiting a nanoscale textured surface wherein the surface includes at least about 60% non-denatured collagen wherein the composition has less than about 6% moisture content is disclosed.

A method of drying bone tissue comprising demineralizing the bone and drying the bone tissue in a solvent at its critical point status is disclosed. In such method, drying using the critical point solvent is done to less than about 6% moisture content.

In one embodiment, a method for producing a demineralized bone matrix composition is provided. Bone is provided, the bone is demineralized, the bone is dried such that collagen fibrils on a surface of the bone are compromised, and nanostructures are added to the bone to provide a nanoscale textured surface. The method of the present invention is set forth in the accompanying claims. A demineralized bone matrix composition produced according to the method of the invention is also provided.

This application refers to various patents, patent applications, journal articles, and other publications: U.S. provisional patent application 60/957,614; PCT/US04/43999; PCT/US05/003092; US Patent No. 7,163,691; PCT/US02/32941; Current Protocols in Molecular Biology, Current Protocols in Immunology, Current Protocols in Protein Science, and Current Protocols in Cell Biology, John Wiley & Sons, N.Y., edition as of July 2002; Sambrook, Russell, and Sambrook, Molecular Cloning: A Laboratory Manual, 3rd ed., Cold Spring Harbor Laboratory Press, Cold Spring Harbor, 2001; Rodd 1989 "Chemistry of Carbon Compounds," vols. 1-5 and Supps, Elsevier Science Publishers, 1989; "Organic Reactions," volumes 1-40, John Wiley and Sons, New York, NY, 1991; March 2001, "Advanced Organic Chemistry," 5th ed. John Wiley and Sons, New York, NY. In the event of a conflict between the specification and any of the references, the specification shall control. Where numerical values herein are expressed as a range, endpoints are included.

### BRIEF DESCRIPTION OF THE DRAWINGS

Figure 1a illustrates the exterior surface of lyophilized demineralized bone matrix as commonly produced in the art.
Figure 1b illustrates the interior of lyophilized demineralized bone matrix as commonly produced in the art.
Figure 2 illustrates the smooth and solid surface of lyophilized demineralized bone matrix fibers.
Figure 3 illustrates the textured surface of critical-point-dried demineralized bone matrix fibers, in accordance with one embodiment.
Figure 4 illustrates the textured surface of demineralized bone matrix wherein the bone has been demineralized, lyophilized, and then milled in dry conditions, in accordance with one embodiment.
Figure 5a illustrates demineralized bone matrix wherein the bone has been demineralized, milled in wet conditions, and then critical point dried, in accordance with one embodiment.
Figure 5b illustrates the textured surface of demineralized bone matrix of Figure 5a.
Figure 6a illustrates demineralized bone matrix wherein the bone has been demineralized, pressed, and then critical point dried, in accordance with one embodiment.
Figure 6b illustrates textured surface of the demineralized bone matrix of Figure 6a.
Figure 7a illustrates collagen coated lyophilized demineralized bone matrix fibers, in accordance with one embodiment.
Figure 7b illustrates the textured surface of the demineralized bone matrix fibers of Figure 7a.
Figure 8a illustrates poly(lactide) coated lyophilized demineralized bone matrix fibers, in accordance with one embodiment.
Figure 8b illustrates the textured surface of the demineralized bone matrix fibers of Figure 8a.

### DEFINITIONS

*Bioactive Agent* or *Bioactive Compound,* as used herein, refers to a compound or entity that alters, inhibits, activates, or otherwise affects biological or chemical events. For example, bioactive agents may include, but are not limited to, osteogenic or chondrogenic proteins or peptides, anti-AIDS substances, anti-cancer substances, antibiotics, immunosuppressants, anti-viral substances, enzyme inhibitors, hormones, neurotoxins, opioids, hypnotics, anti-histamines, lubricants, tranquilizers, anti-convulsants, muscle relaxants and anti-Parkinson substances, anti-spasmodics and muscle contractants including channel blockers, mitotics and anti-cholinergics, anti-glaucoma compounds, anti-parasite and/or anti-protozoal compounds, modulators of cell-extracellular matrix interactions including cell growth inhibitors and antiadhesion molecules, vasodilating agents, inhibitors of DNA, RNA or protein synthesis, anti-hypertensives, analgesics, anti-pyretics, steroidal and non-steroidal anti-inflammatory agents, anti-angiogenic factors, angiogenic factors, anti-secretory factors, anticoagulants and/or antithrombotic agents, local anesthetics, ophthalmics, prostaglandins, anti-depressants, antipsychotic substances, anti-emetics, and imaging agents. In certain embodiments, the bioactive agent is a drug. In some embodiments, the bioactive agent is a growth factor, cytokine, extracellular matrix molecule or a fragment or derivative thereof, for example, a cell attachment sequence such as RGD the abbreviation for the amino acid sequence Arginine-Glycine-Aspartic acid. A more complete listing of bioactive agents and specific drugs suitable for use in the present invention may be found in "Pharmaceutical Substances: Syntheses, Patents, Applications" by Axel Kleemann and Jurgen Engel, Thieme Medical Publishing, 1999; the "Merck Index: An Encyclopedia of Chemicals, Drugs, and Biologicals", edited by Susan Budavari et al., CRC Press, 1996; and the United States Pharmacopeia-25/National Formulary-20, published by the United States Pharmcopeial Convention, Inc., Rockville MD, 2001.

*Biocompatible,* as used herein, describes materials that, upon administration *in vivo,* do not induce undesirable long-term effects.

*Bone,* as used herein, refers to bone that is cortical, cancellous or cortico-cancellous of autogenous, allogenic, xenogenic, or transgenic origin.

*Demineralized,* as used herein, refers to any material generated by removing mineral material from tissue, *e.g.,* bone tissue. The demineralized compositions described herein may include preparations containing less than 5% calcium. The demineralized compositions may comprise less than 1% calcium by weight. Partially demineralized bone is intended to refer to preparations with greater than 5% calcium by weight but containing less than 100% of the original starting amount of calcium. Demineralized bone may have less than 95% of its original mineral content. "Demineralized" is intended to encompass such expressions as "substantially demineralized," "partially demineralized," "surface demineralized," and "fully demineralized." "Partially demineralized" is intended to encompass "surface demineralized."

*Demineralized bone matrix (DBM),* as used herein, refers to any material generated by removing mineral material from bone tissue. The DBM compositions as used herein may include preparations containing less than 5% calcium and, in some embodiments, less than 1% calcium by weight. The DBM compositions may comprise partially demineralized bone (*e.g.,* preparations with greater than 5% calcium by weight but containing less than 100% of the original starting amount of calcium).

*Nanofibers,* as used herein, refer generally to fibers of a submicron level. The fibers may generally be any fibers having at least one side or dimension at or below 1000 nanometers. The fibers may have at least one side or dimension at or below 100 nanometers.

*Nanostructures,* as used herein, refers to structures including, at least, nanofibers, nanoparticles, nanospheres, nanomicelles, and nano-roughness on surfaces. Nanostructures include structures ranging from approximately 1 nm to approximately 1000 nm in at least one dimension.

*Osteoconductive,* as used herein, refers to the ability of a substance to serve as a template or substance along which bone may grow.

*Osteogenic,* as used herein, refers to materials containing living cells capable of differentiation into bone tissue.

*Osteoimplant,* as used herein, refers to any implant prepared in accordance with the disclosures herein and therefore may include expressions such as bone membrane, bone graft, etc.

*Osteoinductive,* as used herein, refers to the quality of being able to recruit cells from the host that have the potential to stimulate new bone formation. Any material that can induce the formation of ectopic bone in the soft tissue of an animal is considered osteoinductive. For example, most osteoinductive materials induce bone formation in athymic rats when assayed according to the method of Edwards et al., "Osteoinduction of Human Demineralized Bone: Characterization in a Rat Model," Clinical Orthopaedics & Rel. Res., 357:219-228, December 1998. In other instances, osteoinduction is considered to occur through cellular recruitment and induction of the recruited cells to an osteogenic phenotype. *Osteoinductivity* score refers to a score ranging from 0 to 4 as determined according to the method of Edwards *et al.* (1998) or an equivalent calibrated test. In the method of Edwards *et al.,* a score of "0" represents no new bone formation; "1" represents 1%-25% of implant involved in new bone formation; "2" represents 26-50% of implant involved in new bone formation; "3" represents 51%-75% of implant involved in new bone formation; and "4" represents >75% of implant involved in new bone formation. In most instances, the score is assessed 28 days after implantation. However, the osteoinductivity score may be obtained at earlier time points such as 7, 14, or 21 days following implantation. In these instances it may be desirable to include a normal DBM control such as DBM powder without a carrier, and if possible, a positive control such as BMP. Occasionally osteoinductivity may also be scored at later time points such as 40, 60, or even 100 days following implantation. Percentage of osteoinductivity refers to an osteoinductivity score at a given time point expressed as a percentage of activity, of a specified reference score. Osteoinductivity may be assessed in an athymic rat or in a human. Generally, as discussed herein, an osteoinductive score is assessed based on osteoinductivity in an athymic rat.

*Superficially demineralized,* as used herein, refers to bone-derived elements possessing at least about 90 weight percent of their original inorganic mineral content, the expression "partially demineralized" as used herein refers to bone-derived elements possessing from about 8 to about 90 weight percent of their original inorganic mineral content and the expression "fully demineralized" as used herein refers to bone containing less than 8% of its original mineral context.

*Textured surface,* as used herein, refers to a surface having a nano-scale texture such as containing nanostructures and other attributes.

### DETAILED DESCRIPTION

### I. Introduction

Bone matrix compositions and, more specifically, bone matrix compositions having nano-scale textured surfaces attractive to cells, and methods for their production are provided. Nano-scale textured surfaces provided on a bone matrix aid in growth factor retention, remodeling, cell attachment, and osteoconductivity of the bone matrix. While specific discussion is made of retaining (throughout processing) and/or imparting textured surfaces, it is to be appreciated that the teachings herein may be applied to non-demineralized bone, partially demineralized bone, or surface demineralized bone. In various embodiments, the nano-scale textured surface may comprise nanofibers, nanoparticles, nanospheres, nanomicelles, or other nano-scale structures on a surface of the bone matrix. In embodiments wherein the nanostructures of the textured surface comprise nanofibers, the nanofibers may be oriented. In some embodiments, the nanostructures of the textured surface may be biologically active. For example, the nanostructure may comprise biologically active biomolecules or incorporated with other biological factors such as peptides, growth factors, cytokines, DNA, RNA, siRNA etc.

Bone naturally has a textured surface. Scanning Electron Microcopy (SEM) has facilitated the study of surface morphology in biological applications. The textured surface generally results from fibrillar collagen, the major organic extracellular matrix (ECM) component of bone. The textured surface is thought to be attractive to cells and to facilitate, at least, cell adhesion. The interactions between cells and bone first occur at the surface of the bone. Collagen (type I) is the major component of DBM and is organized in fibrillar bundles in bone. The fibrillar structure of collagen has been demonstrated to be important for cell attachment, proliferation and differentiation. Collagen fiber bundles vary in diameter from 50 to 500 nanometers and the nanometric fibrillar structure of collagen bundles facilitates cellular recognition.

Methods for retaining the nanoscale textured surface of bone while preparing the bone for implantation in accordance with current standards are provided. Methods for imparting a nanoscale textured surface to bone wherein such surface has been compromised or destroyed are also provided.

To prepare bone for implantation, the bone matrix is typically treated to clean, defat, sterilize, virally inactivate, disinfect, demineralize, dehydrate, and/or dry the bone matrix. Reference is made to U.S. Patent No. 5,846,484, for a description of example treatment of bone intended for implantation. Some treatment processes, while beneficial for some purposes, generally work against conserving or retaining properties of bone. Generally, some of the treatment processes may compromise collagen fibrils on the surface of the bone.

Removal of excess moisture from bone reduces its antigenicity and is done to store and maintain the DBM in active condition for implantation. According to the American Association of Tissue Banks, whole bone containing no more than 6% moisture can be stored at ambient temperatures for up to five years after processing. Typical processes for drying bone include, for example, lyophilization or solvent drying. Typical processes for drying bone such as these, however, generally result in loss of the textured surface of the bone. More specifically, during phase change of the moisture, surface tension of the bone is disturbed. These lead to denaturing of the collagen on the surface of the bone. As a result, dried bone typically exhibits a smooth surface comprising a smear surface of, among other things, denatured collagen fibrils. The collagen fibrils are generally no longer intact and do not retain secondary and tertiary surfaces. Accordingly, the collagen fibrils on the surface of the bone may be considered compromised. The smooth denatured surface does not exhibit the cell attractive qualities as the original nanoscale textured surface of bone.

With specific reference to lyophilization, this process (freeze-drying, i.e., freezing, then sublimation of moisture) is commonly performed on bone to permit its shelf storage for up to several years without spoilage. Lyophilization typically involves freezing whole bone to temperatures as low as -70° C prior to its packaging and storage. While lyophilization is thought to not disrupt physical properties of bone, it does adversely affect biomechanical properties of the bone as well as the nanoscale texture on the surface of the bone. Lyophilization can result in damage to the bone due to dimensional changes that occur during the freezing and dehydrating operations. The adverse mechanical changes have previously been thought to be associated with structural damage occurring in the bone matrix, such as ultrastructural cracks along the collagen fibers. Examination of lyophilized DBM products has revealed that the nanoscale textured surface of the bone is lost at some point during processing. More specifically, while the bone has nanofibrous interiors, the nanoscale texture of the surfaces of the bone is typically destroyed.

Loss of the nanoscale textured surface of the bone may impact the growth factor retention, remodeling, cell attachment, and osteoconductivity of the bone. Providing a nanoscale textured surface to DBM causes the bone morphology to more closely mimic the natural surface of bone or bone extracellular matrix. In some embodiments, a DBM structure having a biomimetic ECM or having an imparted nanoscale textured surface is provided. In other embodiments, a DBM structure having a nanoscale textured surface maintained during processing for implantation is provided. Providing, or retaining, a nanoscale textured surface on DBM increases the functional surface area of the DBM. Increasing the functional surface area of the DBM provides increased surface area to which proteins may adsorb and cells may attach. Anchorage-dependent cells are cells requiring a solid substratum for growth. Anchorage-dependent cells do not grow, for example, in suspension cultures. With improved cell attachment observed to nanofibrous surfaces, providing a nanoscale textured surface to DBM may affect cell proliferation and differentiation. For example, such surface may enhance cell proliferation and differentiation for anchorage-dependent cells such osteoblast-like cells, pre-osteoblastic cells, and mesenchymal stem cells. Providing a nanoscale textured surface to DBM may affect kinetics and affinity for protein release and binding. Further, it is thought that providing a nanoscale textured surface to DBM may have a positive effect on osteoinductivity.

Bone matrix compositions having a nanoscale textured surface are disclosed and methods for their production are thus provided. In some embodiments, the bone is processed in a manner so as to expose or maintain the nanofibrous structure of the bone. In some embodiments, the bone matrix has no smear layer or a smear layer of lesser dimensions than would be expected when the bone matrix is processed using lyophilization. In some embodiments, collagen on the surface of the prepared bone exhibits secondary and tertiary surfaces. In other embodiments, nanoscale textured surfaces are imparted to bone matrix compositions wherein the nanoscale textured surface of the bone has been compromised. Generally, these methods may be applied to mineralized or demineralized bone including partially or surface demineralized bone. Further, while specific discussion may be made to particulated bone, it is to be appreciated that the methods disclosed herein may alternatively be applied to monolithic bone.

In some embodiments, biological activities of the bone matrix may be increased. Accordingly, the bone matrix, and compositions formed from the bone matrix, may variously be referred to as biologically active and/or, in some cases, osteoinductive. The biological activities of the bone composition provided herein that may be increased include but are not limited to osteoinductive activity, osteogenic activity, chondrogenic activity, wound healing activity, neurogenic activity, contraction-inducing activity, mitosis-inducing activity, differentiation-inducing activity, chemotactic activity, angiogenic or vasculogenic activity, exocytosis or endocytosis-inducing activity, or other cell or biological activity. It will be appreciated that bone formation processes frequently include a first stage of cartilage formation that creates the basic shape of the bone, which then becomes mineralized (endochondral bone formation). Thus, in many instances, chondrogenesis may be considered an early stage of osteogenesis, though of course it may also occur in other contexts.

### II. Providing Demineralized Bone

Bone used in the methods described herein may be autograft, allograft, or xenograft. In various embodiments, the bone may be cortical bone, cancellous bone, or cortico-cancellous bone. While specific discussion is made herein to demineralized bone matrix, bone matrix treated in accordance with the teachings herein may be non-demineralized, demineralized, partially demineralized, or surface demineralized. The following discussion applies to demineralized, partially demineralized, and surface demineralized bone matrix.

Any suitable manner of demineralizing the bone may be used. For example, U.S. Patent 5,405,390 describes suitable methods. DBM includes the collagen matrix of the bone together with acid insoluble proteins including bone morphogenic proteins (BMPs) and other growth factors. It can be formulated for use as granules, gels, sponge material or putty and can be freeze-dried for storage. Sterilization procedures used to protect from disease transmission may reduce the activity of beneficial growth factors in the DBM. DBM provides an initial osteoconductive matrix and exhibits a degree of osteoinductive potential, inducing the infiltration and differentiation of osteoprogenitor cells from the surrounding tissues.

DBM preparations have been used for many years in orthopedic medicine to promote the formation of bone. For example, DBM has found use in the repair of fractures, in the fusion of vertebrae, in joint replacement surgery, and in treating bone destruction due to underlying disease such as rheumatoid arthritis. DBM is thought to promote bone formation *in vivo* by osteoconductive and osteoinductive processes. The osteoinductive effect of implanted DBM compositions is thought to result from the presence of active growth factors present on the isolated collagen-based matrix. These factors include members of the TGF-β, IGF, and BMP protein families. Particular examples of osteoinductive factors include TGF-β, IGF-1, IGF-2, BMP-2, BMP-7, parathyroid hormone (PTH), and angiogenic factors. Other osteoinductive factors such as osteocalcin and osteopontin are also likely to be present in DBM preparations as well. There are also likely to be other unnamed or undiscovered osteoinductive factors present in DBM.

In one suitable demineralization procedure, the bone is subjected to an acid demineralization step followed by a defatting/disinfecting step. The bone is immersed in acid to effect demineralization. Acids that can be employed in this step include inorganic acids such as hydrochloric acid and as well as organic acids such as formic acid, acetic acid, peracetic acid, citric acid, propionic acid, *etc.* The depth of demineralization into the bone surface can be controlled by adjusting the treatment time, temperature of the demineralizing solution, concentration of the demineralizing solution, agitation intensity during treatment, and other applied forces such as vacuum, centrifuge, pressure, and other factors such as known to those skilled in the art. Thus, in various embodiments, the DBM may be fully demineralized, partially demineralized, or surface demineralized.

The demineralized bone is rinsed with sterile water and/or buffered solution(s) to remove residual amounts of acid and thereby raise the pH. A suitable defatting/disinfectant solution is an aqueous solution of ethanol, the ethanol being a good solvent for lipids and the water being a good hydrophilic carrier to enable the solution to penetrate more deeply into the bone particles. The aqueous ethanol solution also disinfects the bone by killing vegetative microorganisms and viruses. Ordinarily, at least about 10 to 40 percent by weight of water (*i.e.,* about 60 to 90 weight percent of defatting agent such as alcohol) is present in the defatting disinfecting solution to produce optimal lipid removal and disinfection within a given period of time. A suitable concentration range of the defatting solution is from about 60 to about 85 weight percent alcohol, or about 70 weight percent alcohol.

In some embodiments, the demineralized bone may be further treated to affect properties of the bone. For example, the DBM may be treated to disrupt the collagen structure of the DBM. Such treatment may comprise collagenase treatment, heat treatment, mechanical treatment, or other. Reference is made to U.S. Provisional Patent Applications 60/944,408, 60/944,417, and 60/957,614 for further treatment options. In embodiments wherein collagen disruption affects the nanoscale textured surface, such nanoscale textured surface may later be imparted to the bone.

While demineralized bone is specifically discussed herein, in some embodiments, the teachings herein may be applied to non-demineralized bone, to partially demineralized bone, or to surface demineralized bone.

### III. Provide Bone Particles

The bone may be particulated. If the bone is demineralized, the bone may be particulated before, during or after demineralization. As previously discussed, in some embodiments, the bone may be monolithic and may not be particulated. Accordingly, while specific discussion is given to particulating bone, the methods disclosed herein and the nanoscale textured surfaces disclosed herein may be used with monolithic bones or implants, including, for example, surface demineralized implants or fully demineralized cortical bone implants.

The bone may be milled and ground or otherwise processed into particles of an appropriate size before or after demineralization. The particles may be particulate or fibrous. The terms milling or grinding are not intended to be limited to production of particles of a specific type and may refer to production of particulate or fibrous particles. In certain embodiments, the particle size may be greater than 75 microns, such as ranging from about 100 to about 3000 microns, or from about 200 to about 2000 microns. After grinding, the bone particles may be sieved to select those particles of a desired size. In certain embodiments, the particles may be sieved though a 50 micron sieve, a 75 micron sieve, or a 100 micron sieve.

### IV. Dry Bone Matrix

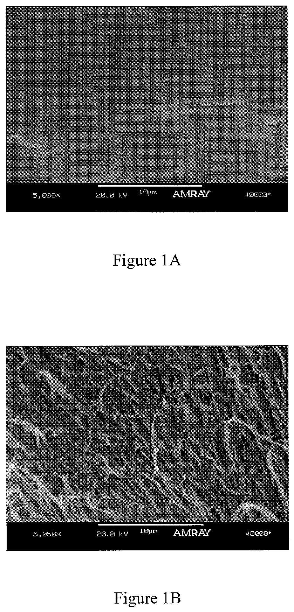

As previously discussed, DBM typically is dried, for example via lyophilization or solvent drying, to store and maintain the DBM in active condition for implantation. As noted, however, each of these processes is thought to destroy the nanofibrous surface structure of bone. Figures 1a and 1b illustrate differences in the smooth exterior surface and the textured interior surface of lyophilized bone matrix. Figure 1a illustrates the smooth surface of the exterior surface of lyophilized bone matrix while Figure 1b illustrates the nanofibrous interior surface of lyophilized bone matrix. As may be appreciated, the structural damage of the exterior surface, specifically the loss of the nanofibrous nature of the surface, reduces the overall surface area. Physical alterations to the surface and reduction in surface area can affect cell attachment, mobility, proliferation, and differentiation. The surface's affinity for growth factors and release kinetics of growth factors from the surface may also be altered.

Accordingly, in some embodiments, methods for drying bone to store and maintain the bone in active condition for implantation that maintain a nanoscale textured surface on an exterior surface of the bone are provided. In one embodiment, the bone matrix is treated using critical point drying (CPD) technique, thereby reducing destruction of the nanoscale textured surface of the bone. While specific description is made to critical point drying, it is to be appreciated that, in alternative embodiments, super critical point treatment may be used. In various embodiments utilizing CPD, a percentage of collagen fibrils on the surface of the bone are non-denatured after drying to a residual moisture content of approximately 15% or less. In some embodiments, after drying, the bone matrix has a residual moisture content of approximately 8% or less. In some embodiments, after drying, the bone matrix has a residual moisture content of approximately 6% or less. In some embodiments, after drying, the bone matrix has a residual moisture content of approximately 6% or less. In some embodiments, after drying, the bone matrix has a residual moisture content of approximately 3% or less. In some embodiments, after drying, the bone matrix has a residual moisture content of approximately 1% or less. In some embodiments, approximately 60% or more of the collagen fibrils on the surface of the bone matrix are non-denatured. In some embodiments, approximately 75% or more of the collagen fibrils on the surface of the bone matrix are non-denatured. In some embodiments, approximately 90% or more of the collagen fibrils on the surface of the bone matrix are non-denatured. Collagen fibrils typically range in size from approximately 50 nm to approximately 500 nm. In some embodiments, bone matrices processed using CPD retain a nanoscale texture surface having surface features on the order of approximately 1 nm to approximately 1000 nm. In certain embodiments, bone matrices processed using CPD retain a nanoscale texture surface having surface features on the order of approximately 40 to approximately 600 nm. In other embodiments, bone matrices processed using CPD retain a nanoscale texture surface having surface features on the order of approximately 50 to approximately 500 nm. In some embodiments, nanostructures are imparted to bone matrices processed using CPD and retaining a nanoscale texture surface, thus providing an enhanced nanoscale texture surface on the bone matrix, as described below.

Evaporative drying and freeze drying of specimens can cause deformation and collapse of structures, especially sub-micrometric and nanometric structures. Without wishing to be bound to a particularly theory, this deformation and structure is thought to be caused because, as a substance crosses the boundary from liquid to gas, the substance volatilizes such that the volume of the liquid decreases. As this happens, surface tension at the solid-liquid interface pulls against any structures to which the liquid is attached. Delicate structures, such as the nanofibrous structures of bone surfaces, tend to be broken apart by this surface tension. Such damage may be caused by the effects of surface tension on the liquid/gas interface. Critical point drying is a technique that avoids effects of surface tension on the liquid/gas interface by substantially preventing a liquid/gas interface from developing. Critical point or supercritical drying does not cross any phase boundary, instead passing through the supercritical region, where the distinction between gas and liquid ceases to apply. As a result, materials dried using critical point drying are not exposed to damaging surface tension forces. When the critical point of the liquid is reached, it is possible to pass from liquid to gas without abrupt change in state. Critical point drying can be used with bone matrices to phase change from liquid to dry gas without the effects of surface tension. Accordingly, bone dehydrated using critical point drying can retain at least some of the nanoscale texture on the surface.

In some embodiments, critical point drying is carried out using carbon dioxide. However, other mediums such as freon, including Freon 13 (chlorotrifluoromethane), may be used. Generally, fluids suitable for supercritical drying include carbon dioxide (critical point 304.25 K at 7.39 MPa or 31.1 °C at 7.39 MPa (1072 psi) or 31.2 °C and 73.8 MPa (73.8 bar) and freon (∼300 K at 3.5-4 MPa or 25 to 30°C at 3.45 to 4.14 MPa (500-600 psi)). Nitrous oxide has similar physical behavior to carbon dioxide, but is a powerful oxidizer in its supercritical state. Supercritical water is also a powerful oxidizer, partly because its critical point occurs at such a high temperature (374°C) and pressure 22.064MPa (3212 psi / 647K).

In some embodiments, the bone may be pretreated to remove water prior to critical point drying. Thus, in accordance with one embodiment, bone matrix is dried using carbon dioxide in (or above) its critical point status. After demineralization, bone matrix samples (in water) may be dehydrated to remove residual water content. Such dehydration may be, for example, through a series of graded ethanol solutions (for example, 20%, 50%, 70%, 80%, 90%, 95%, 100% ethanol in deionized water). In some embodiments, penetrating the tissue with a graded series of ethanol solutions or alcohols may be accomplished in an automated fashion. For example, pressure and vacuum could be used to accelerate penetration into the tissue. U.S. Patent No. 6,162,258 discusses processes for penetrating tissue. Such solvent drying may be done to an extent wherein the nanoscale textured surface of the bone is not disrupted or such that at least some percentage of collagen fibrils on the surface remain non-denatured.

In alternative embodiments, other means or procedures for removing water (drying or dehydrating) from the bone may be used. For example, the bone may be washed with other dehydrating liquids such as acetone to remove water, exploiting the complete miscibility of these two fluids. The acetone may then washed away with high pressure liquid carbon dioxide.

In some embodiments, the dehydrated bone matrix is placed in a chamber within a critical point drying (CPD) apparatus and flushed with liquid CO₂ to remove ethanol (or other dehydrating liquid). Flushing with liquid CO₂ may be done one or more times. The temperature and/or pressure are then raised to the critical point (the critical point for CO₂ is reached at 31.2 °C and 73.8 MPa (73.8 bar)). To perform critical point drying, the temperature and pressure may continue to be raised, for example to 40°C with corresponding pressure of 85 MPa (85 bar). Thus, in some embodiments, the liquid carbon dioxide is heated until its pressure is at or above the critical point, at which time the pressure can be gradually released, allowing the gas to escape and leaving a dried product.

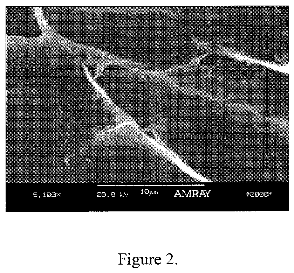

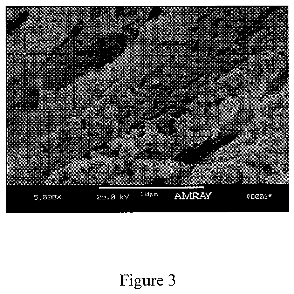

Figure 2 illustrates the surface of lyophilized DBM fibers. As shown, the surface is generally smooth and solid. Figure 3 illustrates the surface of critical point dried DBM fibers, in accordance with one embodiment. As shown, the surface has a nanoscale texture comprising nanofibers and the nanofibers are oriented. DBM dried with critical point carbon dioxide has increased biological activity and osteoinductivity compared to DBM dried with lyophilization.

In a further embodiment, the critical point dried samples may further be treated, or alternatively be treated, with supercritical carbon dioxide (carbon dioxide above the critical point). Supercritical CO₂ may also be useful in viral inactivation. In some embodiments, thus, the bone matrix is placed in a supercritical CO₂ chamber and liquid CO₂ is introduced, for example, by an air pump. The temperature is raised to 105°C with corresponding pressure about 485 PMa (485 bar). In alternative embodiments, other temperatures and/or pressures above the critical point of CO₂ may be used. The samples are soaked in supercritical CO₂ for a certain time and CO₂ is released. The resulting bone samples retain nanofibrous surface morphologies and osteoinductivity after such treatment.

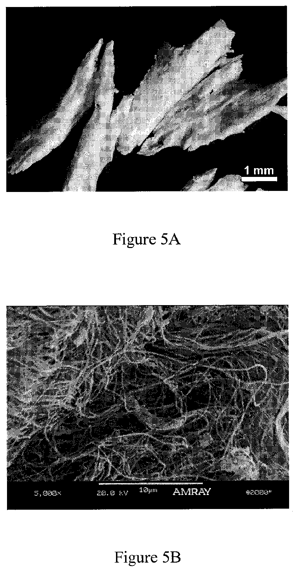

In yet a further embodiment, monolithic bone is demineralized and particulated before drying. Accordingly, the bone may be demineralized in monolithic pieces. The demineralized monolithic pieces may then be milled in a wet condition and critical point dried, for example using carbon dioxide as a medium. Figures 5a and 5b illustrate demineralized bone fibers and their nanofibrous surface structures, respectively, of demineralized bone matrix fiber processed in accordance with this embodiment.

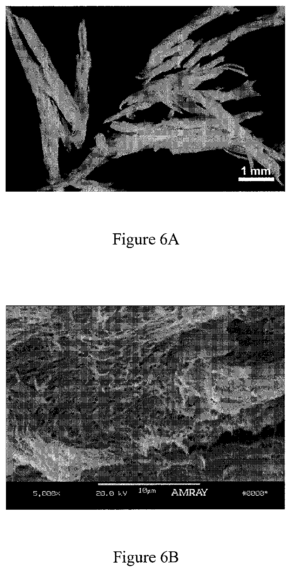

In yet a further embodiment, monolithic bone is demineralized and dried before particulating (if done). Accordingly, the bone may be demineralized in monolithic pieces. The DBM is pressed in a wet condition and then critical point dried, for example using carbon dioxide as a medium. Figures 6a and 6b illustrate demineralized bone matrix fibers and their nanofibrous surface structures, respectively, prepared in accordance with this embodiment. In alternatives of this embodiment, the demineralized and dried monolithic bone is not particulated and is processed as a monolithic implant.

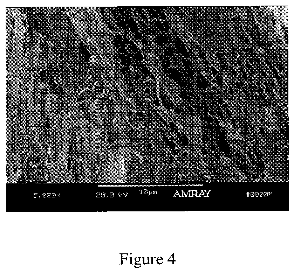

As noted, lyophilization and other methods of drying bone destroy the nanoscale texture on the exterior surface of the bone. The interior of the bone, however, may retain nanoscale texture. Thus, in some embodiments, methods disclosed herein expose the nanoscale texture in the interior of lyophilized, demineralized bone matrix. Specifically, in such embodiments, monolithic bone matrix may be demineralized and lyophilized. The demineralized, lyophilized bone matrix is then milled to obtain demineralized particles from the interior of the bone matrix. Figure 4 illustrates surface of such processed demineralized bone matrix. As shown, the surface of the bone particles is nanofibrous. Thus, this embodiment exposes interior nanofibrous structures of a demineralized monolithic bone matrix to surface after milling. Milling may be done to particulate the bone in a fibrous manner or in a particle manner.

In other embodiments, the bone matrix is dried using lyophilization and nanoscale textured surface is imparted to the bone matrix as described below.

In accordance with various embodiments, the bone compositions provided herein may have neutral and polar lipid content below approximately 5% before defatting and demineralizing.

### V. Impart Nanofibrous Structure to Bone Matrix

Nanostructures are imparted to the bone to provide or enhance a nanoscale textured surface. Thus, for example, the bone matrix may be processed in a typical manner wherein the nanoscale textured surface is compromised and nanostructures may be imparted to the bone matrix. In other embodiments, bone matrix having a nanoscale textured surface, such as bone matrix processed using CPD, may have further nanostructures imparted thereto to enhance the nanoscale textured surface.

Nanostructures are imparted to the bone matrix, collagen, polylactide nanostructures, or other nanostructure materials may be added to the surface of bone matrix. Such nanostructures include nanofibers, nanoparticles, nanospheres and nanomicelles. The nanostructures can be prepared before or during incorporation process. Associating the nanostructure materials with the bone matrix is selected from adsorption, coating, precipitating self-assembly, and electrospinning.

In some embodiments, nanostructure materials added to the bone matrix may contain therapeutic factors that can promote cellular response and tissue regeneration or have other functional properties. The factors can be antibiotics, functional peptides, proteins, growth factors, cytokines, DNAs, RNAs, siRNAs and the combination of those.

In one embodiment, imparting nanostructures to bone matrix may be done by coating or precipitating a nanostructure material on the bone matrix, thereby providing or enhancing a nanoscale textured surface of the bone matrix. In various embodiments the nanostructure material may be naturally occurring or may be synthetic or may be a blend or mixture of naturally occurring and synthetic materials. Suitable naturally occurring materials include, for example, collagen, gelatin, alginate, hyaluronic acid, chitosan, other extracellular matrix components, and tissue extracts. Suitable synthetic materials include, for example, polymeric materials, bone minerals, apatite, hydroxyapatite, bioglass, and bioceramic-biopolymer nanocomposites. A further suitable material may be, for example, hydroxyapatite/chitosan (HAp/CTS). The nanostructure may have a uniform structure, a nonuniform structure, a fibrous structure, a nanoporous structure, a nano-scale roughness, or other structure. In some embodiments, the nanostructures may have a diameter of approximately 1-2 µm. In various embodiments, the nanostructure may be approximately 1 nm to approximately 1000 nm in at least one dimension. For example, the nanostructure may be approximately 40 nm to approximately 600 nm, from 50 nm to approximately 500 nm, from approximately 100 to approximately 400 nm, or other suitable size.

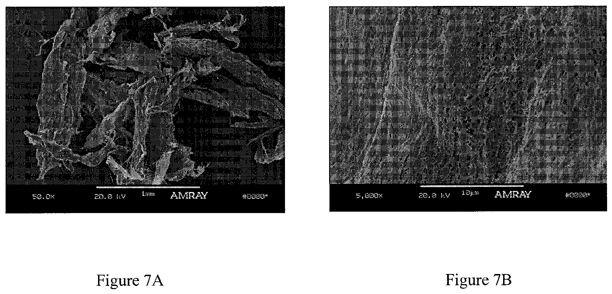

Thus, in some embodiments, naturally derived biomacromolecules such as collagen and gelatin may be used to form a nanoscale textured surface on the DBM. The nanostructure material may be imparted to, or placed on, the bone matrix in any suitable manner. In some embodiments, the DBM is mixed with a collagen or gelatin solution. The mixture is gelled and dried using lyophilization or critical point drying techniques. The resulting DBM can be cross-linked, for example using a chemical such as glutaraldehyde. Properties of the nanoscale textured surface provided by the nanostructures may be adjusted, for example by concentration of polymer, gelation conditions (temperature, pH, solvent), and cross-linking parameters. Figure 7 illustrates demineralized bone matrix fibers and their nanofibrous surface, respectively, of DBM fibers wherein nanofibrous collagen has been applied to the DBM by coating/precipitation, in accordance with one embodiment.

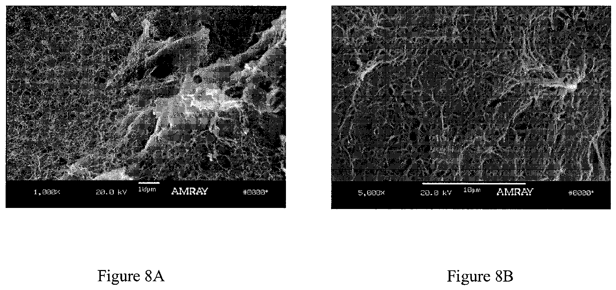

In another embodiment not being part of the invention, imparting a nanoscale textured surface to bone matrix may be done by phase separation of a synthetic polymer. A typical biodegradable synthetic polymer, poly(L-lactide), is phase separated in tetrahydrofuran (THF) to introduce a nanofibrous coating on DBM. The phase separation temperature and polymer concentration are varied to adjust the density and morphology of the polymer surface coating. The polymer coating process can be performed either on typically-processed DBM having a compromised surface (e.g., lyophilized and having a smear layer and/or no nanoscale textured on the surface) or DBM with a nanoscale textured surface (e.g., critical point dried). Figures 8a and 8b illustrate DBM fibers and their surface, respectively, after adding nanofibrous poly(L-lactide) by coating and phase separation techniques. Adding nanostructures to bone matrix substantially increase the surface area of the bone matrix. Such increased surface area may facilitate regulation of protein adsorption and cellular activity.

In yet other embodiments, imparting a nanoscale textured surface to bone matrix may be done by electrospinning nanofibers, such as polymer nanofibers, onto a DBM surface. In an example embodiment electrospinning of nanofibers, a high voltage is applied to a polymer solution, which overcomes the surface tension to form a charged jet. The charged polymer solution is ejected, dried, and solidified onto a grounded substrate, in this case, a DBM substrate in thin sheet form. The ejected polymer solutions repel each other to form thin fibers. By controlling the spinning conditions, the resulting fibers can range from about 0.02 µm to about 20 µm. A variety of polymers can be electrospun onto DBM surface. The polymers include biodegradable synthetic polymers such as poly(lactide) (PLLA), poly(lactide-co-glycolide) (PLGA), polycaprolactone (PCL), poly(L-lysine), etc. and naturally derived polymers such as collagen, gelatin, chitosan, hyaluronic acid, alginate, etc.

In yet other embodiments, imparting nanostructures to a surface of a bone matrix may be done by self-assembling nanostructures onto the DBM surface. Self-assembly refers to a spontaneous organization of individual molecules into a well-defined structure with noncovalent interactions. DBM comprises collagen and other non-collagenous proteins or biomacromolecules. The biomacromolecules in DBM may be treated to form surfaces with charged cationic (-NH₃⁺) or anionic (-COO⁻) polyelectrolytes or with different hydrophilic and hydrophobic domains. Biomacromolecules with opposite charges or hydrophilic/hydrophobic properties are then immobilized onto the DBM surface. Under certain conditions, the immobilized biomolecules form nanostructures. The biomacromolecules for nanostructure self-assembly can be polypeptides, oligopeptides, peptide-maphiphiles, or synthetic diblock/triblock polymers and dendrimers.

In yet other embodiments, biologically active factors may be incorporated into the nanostructures which are imparted onto the DBM surfaces. The biologically active factors may be compounds with low molecular weight or macromolecules with high molecular weight including proteins, cytokines, growth factors, enzymes, DNA, RNAs, and others. Suitable compounds include, for example, those described with respect to Optional Additives, below. In one embodiment, macromolecules form nanostructures on the surface of the DBM, for example via self-assembly. In another embodiment, the factors are dissolved or dispended in a solution of materials which form nanostructures on the DBM surfaces, for example via electrospinning, coating, precipitation, or other mechanism. In yet another embodiment, factors are encapsulated into polymer nanoparticles using a double emulsion technique wherein biological factors are encapsulated by the polymer nanoparticles. The nanoparticles may then be provided on the DBM, for example via coating. The nanostructure imparted onto the DBM surface provides a controlled delivery approach for the biologically active molecules.

Further, in some embodiments, the embodiments described above may be applied to a bone matrix wherein the nanofibrous structure has not been compromised. For example, a nanofibrous coating may be applied to a bone matrix wherein the nanofibrous structure has not been compromised.

### VI. Whitening or Lightening the Bone Matrix

In accordance with some embodiments, a method, which is not part of the present invention, is disclosed for whitening bone or DBM without exposure to bleach or peroxide. Specifically, CO₂ may be used as a solvent for lipids or lipidphilic substances. Removing lipids or lipidphilic substances from the bone matrix results in a lightened or whitened bone matrix. Thus, in methods using critical point CO₂ for drying the bone matrix, the bone may be whitened.

### VII. Optional Additives

In accordance with various embodiments, the bone matrix provided herein may be used with growth factors, extracts, peptide hormones, or other additives to increase the osteoinductive capacity or that otherwise encourage cell or biological activity of the bone matrix or to impart other benefits to the bone matrix. As may be appreciated, in some embodiments, an additive may be encapsulated in the nanostructure imparted to the bone matrix. It will be appreciated that the amount of additive used will vary depending upon the type of additive, the specific activity of the particular additive preparation employed, and the intended use of the composition. The desired amount is readily determinable by the user.

The nanoscale textured surface of the bone compositions provided herein aids in growth factor retention and controlled release, cell attachment, and osteoconductivity of the bone matrix. It also imparts a larger surface area to the bone. The larger surface area can affect protein adsorption. Further, the interaction between nanofibers and additives affects the release kinetics of additives from matrix. The imparted nanoscale textured surface may provide additional growth factor release mechanism for bone matrix.

Any of a variety of medically and/or surgically useful optional substances can be incorporated in, or associated with, the osteoinductive factors either before, during, or after preparation of the osteoinductive or biologically active composition. Thus, for example when demineralized bone particles are used to form the material, one or more of such substances may be introduced into the demineralized bone particles, for example, by soaking or immersing the bone particles in a solution or dispersion of the desired substance(s).

Medically/surgically useful substances that can be readily combined with the DBM include, for example, collagen, insoluble collagen derivatives, etc., and soluble solids and/or liquids dissolved therein, e.g., antiviricides, particularly those effective against HIV and hepatitis; antimicrobials and/or antibiotics such as erythromycin, bacitracin, neomycin, penicillin, polymyxin B, tetracyclines, viomycin, chloromycetin and streptomycins, cefazolin, ampicillin, azactam, tobramycin, clindamycin and gentamicin, etc.; biocidal/biostatic sugars such as dextroal, glucose, etc.; amino acids, peptides, vitamins, inorganic elements, co-factors for protein synthesis; hormones; endocrine tissue or tissue fragments; synthesizers; enzymes such as collagenase, peptidases, oxidases, etc.; polymer cell scaffolds with parenchymal cells; angiogenic drugs and polymeric carriers containing such drugs; collagen lattices; antigenic agents; cytoskeletal agents; cartilage fragments, living cells such as chondrocytes, bone marrow cells, mesenchymal stem cells, natural extracts, tissue transplants, bone, demineralized bone powder, autogenous tissues such blood, serum, soft tissue, bone marrow, etc.; bioadhesives, bone morphogenic proteins (BMPs), angiogenic factors, transforming growth factor (TGF-beta), insulin-like growth factor (IGF-1); growth hormones such as somatotropin; bone digestors; antitumor agents; immuno-suppressants; permeation enhancers, e.g., fatty acid esters such as laureate, myristate and stearate monoesters of polyethylene glycol, enamine derivatives, alpha-keto aldehydes, etc.; and, nucleic acids. The amounts of such optionally added substances can vary widely with optimum levels being readily determined in a specific case by routine experimentation.

In one embodiment, a tissue-derived extract may be added to the bone matrix. U.S. patent application no. 12/140,044 discloses such extracts and addition of such extracts to DBM. For example, a tissue-derived extract or partially demineralized bone may be added to the bone matrix. The extract may be derived from any suitable tissue, such as bone, bladder, kidney, brain, skin, or connective tissue. Further, the extract may be derived in any suitable manner. The extract may be allogeneic, autogeneic, xenogeneic, or transgenic. In embodiments wherein the extract is bone-derived, the bone may be cortical, cancellous, or corticocancellous and may be demineralized, partially demineralized, or mineralized. In some embodiments, the extract may comprise demineralized bone, partially demineralized bone, mineral derived from bone, or collagen derived from bone. In some embodiments, the tissue-derived extract may be a protein extract.

Bone regeneration involves a multitude of cells (e.g. cartilage, fibroblasts, endothelial, etc.) besides osteoblasts. Stem cells may be combined with the bone matrix. Accordingly, the bone matrix composition may be used to deliver stem cells, which offers the potential to give rise to different types of cells in the bone repair process. The bone matrix composition may further comprise a cell such as an osteogenic cell or a stem cell. The additive may comprise radiopaque substances, angiogenesis promoting materials, bioactive agents, osteoinducing agents, or other. Reference is made to U.S. Patent Application Serial Nos. 11/555,606 and 11/555,608 for specific discussion of possible additives.

In certain embodiments, the additive is adsorbed to or otherwise associated with the bone matrix. The additive may be associated with the bone matrix through specific or nonspecific interactions, or covalent or noncovalent interactions. Examples of specific interactions include those between a ligand and a receptor, an epitope and an antibody, *etc.* Examples of nonspecific interactions include hydrophobic interactions, electrostatic interactions, magnetic interactions, dipole interactions, van der Waals interactions, hydrogen bonding, *etc.* In certain embodiments, the additive is attached to the bone matrix composition, for example, to the carrier, using a linker so that the additive is free to associate with its receptor or site of action *in vivo.* In other embodiments the additive is either covalently or non-covalently attached to the carrier. In certain embodiments, the additive may be attached to a chemical compound such as a peptide that is recognized by the carrier. In another embodiment, the additive is attached to an antibody, or fragment thereof, that recognizes an epitope found within the carrier. In certain embodiments at least additives are attached to the osteoimplant. In other embodiments at least three additives are attached to the osteoinductive or biologically active composition. An additive may be provided within the osteoinductive or biologically active composition in a sustained release format. For example, the additive may be encapsulated within biodegradable polymer nanospheres, microspheres, *etc.*

Any suitable method for adding, or dispersing, the additive to the bone matrix may be used. Generally, the procedures used to formulate or disperse the additive onto the bone matrix are sensitive to the physical and chemical state of both the additive and the bone matrix.

### VIII. Addition of Bone Matrix to Carrier

The bone matrix provided herein may be combined, with or without additives, with a carrier or excipient to achieve consistency for specific uses. For example, a carrier may be selected to provide the bone matrix composition in a gel consistency, a putty consistency, a matrix consistency, or other to form an osteoinductive or biologically active composition. The osteoinductive or biologically active composition may be configured to be moldable, extrudable, or substantially solid. The osteoinductive or biologically active composition may be configured to substantially retain its shape in water for a period of time. The osteoinductive or biologically active composition may form an osteoimplant useful in clinical applications. Suitable carriers may include surface demineralized bone; mineralized bone; nondemineralized cancellous scaffolds; demineralized cancellous scaffolds; cancellous chips; particulate, demineralized, guanidine extracted, species-specific (allogenic) bone; specially treated particulate, protein extracted, demineralized, xenogenic bone; collagen; synthetic hydroxyapatites; synthetic calcium phosphate materials; tricalcium phosphate, sintered hydroxyapatite, settable hydroxyapatite; polylactide polymers; polyglycolide polymers, polylactide-co-glycolide copolymers; tyrosine polycarbonate; calcium sulfate; collagen sheets; settable calcium phosphate; polymeric cements; settable poly vinyl alcohols, polyurethanes; resorbable polymers; and other large polymers; liquid settable polymers; and other biocompatible settable materials. The carrier may further comprise a polyol (including glycerol or other polyhydroxy compound), a polysaccharide (including starches), a hydrogel (including alginate, chitosan, dextran, pluronics, N,O-carboxymethylchitosan glucosamine (NOCC)), hydrolyzed cellulose, or a polymer (including polyethylene glycol). In embodiments wherein chitosan is used as a carrier, the chitosan may be dissolved using known methods including in water, in mildly acidic aqueous solutions, in acidic solutions, etc.

The carrier may further comprise a hydrogel such as hyaluronic acid, dextran, pluronic block copolymers of polyethylene oxide and polypropylene, and others. Suitable polyhodroxy compounds include such classes of compounds as acyclic polyhydric alcohols, nonreducing sugars, sugar alcohols, sugar acids, monosaccharides, disaccharides, water-soluble or water dispersible oligosaccharides, polysaccharides and known derivatives of the foregoing. An example carrier comprises glyceryl monolaurate dissolved in glycerol or a 4:1 to 1:4 weight mixture of glycerol and propylene glycol. Reference is made to U.S. Patent No. 5, 314,476 for other carriers including polyhydroxy carriers, to U.S. Patent No. 6,884,778 for biocompatible macromers that may be used as carriers, and to U.S. Patent Publication No. 2003/0152548 for cross-linkable monomers that may be used as carriers. Settable materials may be used, and they may set up either in situ, or prior to implantation. Optionally, xenogenic bone powder carriers also may be treated with proteases such as trypsin. Xenogenic carriers may be treated with one or more fibril modifying agents to increase the intraparticle intrusion volume (porosity) and surface area. Useful agents include solvents such as dichloromethane, trichloroacetic acid, acetonitrile and acids such as trifluoroacetic acid and hydrogen fluoride. The choice of carrier may depend on the desired characteristics of the composition. In some embodiments, a lubricant, such as water, glycerol, or polyethylene glycol may be added.

Any suitable shape, size, and porosity of carrier may be used. In some embodiments, the carrier may be settable and/or injectable. Such carrier may be, for example, a polymeric cement, a settable calcium phosphate, a settable poly vinyl alcohol, a polyurethane, or a liquid settable polymer. Suitable settable calcium phosphates are disclosed in U.S. Patent Nos. 5,336,264 and 6,953,594. Hydrogel carriers may additionally impart improved spatial properties, such as handling and packing properties, to the osteoconductive composition. An injectable carrier may be desirable where the composition is used with a containment device. In addition, selected materials must be biocompatible in vivo and optionally biodegradable. In some uses, the carrier acts as a temporary scaffold until replaced by new bone. Polylactic acid (PLA), polyglycolic acid (PGA), and various combinations have different dissolution rates in vivo. In bone, the dissolution rates can vary according to whether the composition is placed in cortical or trabecular bone.

The carrier may comprise a shape-retaining solid made of loosely adhered particulate material, e.g., with collagen. It may alternatively comprise a molded, porous solid, a monolithic solid, or an aggregate of close-packed particles held in place by surrounding tissue. Masticated muscle or other tissue may also be used. Large allogenic bone implants may act as a carrier, for example where their marrow cavities are cleaned and packed with DBM and, optionally, the osteoinductive factors.

One way to protect small size particles from cellular ingestion and/or to provide a diffusion barrier is to embed them in a monolithic bioabsorbable matrix, and then fragment the particle-containing monolithic matrix into particle sizes greater than 70 microns, for example, greater than 100 microns, or greater than 150 microns in their smallest dimension. Suitable matrices for embedding DBM compositions include biocompatible polymers and setting calcium phosphate cements. Generally the DBM composition/polymer weight ratio will range from about 1:5 to about 1:3. In the case of calcium phosphate, the DBM may be present up to 75% by weight. In one embodiment, bone matrix is embedded in a resorbable polymer. In a further embodiment, bone matrix particles are embedded in one of the setting calcium phosphates known to the art.

The carrier may comprise a number of materials in combination, some or all of which may be in the form of fibers and/or particles. The carrier may comprise calcium phosphates. Driessens et al. "Calcium phosphate bone cements," Wise, D.L., Ed., Encyclopedic Handbook of Biomaterials and Bioengineering, Part B, Applications New York: Marcel Decker; Elliott, Structure and Chemistry of the Apatites and Other Calcium Phosphates Elsevier, Amsterdam, 1994. Calcium phosphate matrices include, but are not limited to, dicalcium phosphate dihydrate, monetite, tricalcium phospate, tetracalcium phosphate, hydroxyapatite, nanocrystalline hydroxyapatite, poorly crystalline hydroxyapatite, substituted hydroxyapatite, and calcium deficient hydroxyapatites.

The carrier may comprise an osteoinductive material such as a mineralized particulated material, osteoinductive growth factors, or partially demineralized bone. The mineralized particulated material may be TCP, hydroxyapatite, mineral recovered from bone, cancellous chips, cortical chips, surface demineralized bone, or other material. The osteoinductive material may be combined with a further carrier such as starch or glycerol. Accordingly, in some embodiments, the bone matrix may act as a carrier for the tissue-derived extract.

The bone matrix composition may be completely insoluble or may be slowly solubilized after implantation. Following implantation, the composition may resorb or degrade, remaining substantially intact for at least one to seven days, or for two or four weeks or longer and often longer than 60 days. The composition may thus be resorbed prior to one week, two weeks, three weeks, or other, permitting the entry of bone healing cells.

### IX. Formation of an Implant

The bone matrix compositions provided herein may be used to form an osteoinductive or biologically active osteoimplant, such as an implant that has surfaces that encourage cell or biological activity. The osteoimplant resulting from the bone matrix, additive, and/or carrier may be flowable, have a putty consistency, may be shaped or molded, and/or may be deformable. The osteoimplant may assume a determined or regular form or configuration such as a sheet, plate, disk, tunnel, cone, or tube, to name but a few. Prefabricated geometry may include, but is not limited to, a crescent apron for single site use, an I-shape to be placed between teeth for intra-bony defects, a rectangular bib for defects involving both the buccal and lingual alveolar ridges, neutralization plates, reconstructive plates, buttress plates, T-buttress plates, spoon plates, clover leaf plates, condylar plates, compression plates, bridge plates, or wave plates. Partial tubular as well as flat plates can be fabricated from the osteoimplant. Such plates may include such conformations as, e.g., concave contoured, bowl shaped, or defect shaped. The osteoimplant can be machined or shaped by any suitable mechanical shaping means. Computerized modeling can provide for the intricately-shaped three-dimensional architecture of an osteoimplant custom-fitted to the bone repair site with great precision. In embodiments wherein the osteoimplant is shaped or moldable, the implant may retain coherence in fluids.

Accordingly, the osteoinductive or biologically active bone matrix composition may be subjected to a configuring step to form an osteoimplant. The configuring step can be employed using conventional equipment known to those skilled in the art to produce a wide variety of geometries, *e.g.,* concave or convex surfaces, stepped surfaces, cylindrical dowels, wedges, blocks, screws, and the like. A surgically implantable material fabricated from elongated bone particles that have been demineralized according to the invention, which may be shaped as a sheet, and processes for fabricating shaped materials from demineralized bone particles is disclosed in U.S. Patent Nos. 5,507,813 and 6,436,138, respectively. Suitable sheets include those sold under the trade name Grafton® DBM Flex, which must be wetted/hydrated prior to use to be useful for implantation. Such sheets have recently been reported as effective in seeding human bone marrow stromal cells (BMSCs), which may be useful in the repair of large bone defects. Kasten et al., "Comparison of Human Bone Marrow Stromal Cells Seeded on Calcium-Deficient Hydroxyapatite, Betatricalcium Phosphate and Demineralized Bone Matrix," Biomaterials, 24(15):2593-603, 2003. Also useful are demineralized bone and other matrix preparations comprising additives or carriers such as binders, fillers, plasticizers, wetting agents, surface active agents, biostatic agents, biocidal agents, and the like. Some exemplary additives and carriers include polyhydroxy compounds, polysaccharides, glycosaminoglycan proteins, nucleic acids, polymers, polaxomers, resins, clays, calcium salts, and/or derivatives thereof.

The osteoinductive or biologically active bone matrix composition may be placed in a containment device such as a porous mesh to provide a delivery system. The device may comprise a polymer (such as polyalkylenes (e.g., polyethylenes, polypropylenes, etc.), polyamides, polyesters, polyurethanes, poly(lactic acid-glycolic acid), poly(lactic acid), poly(glycolic acid), poly(glaxanone), poly(orthoesters), poly(pyrolicacid), poly(phosphazenes), L-co-G, etc.), other bioabsorbable polymer such as Dacron or other known surgical plastics, a natural biologically derived material such as collagen, a ceramic (with bone-growth enhancers, hydroxyapatite, etc.), PEEK (polyether-etherketone), dessicated biodegradable material, metal, composite materials, a biocompatible textile (e.g., cotton, silk, linen), or other. The containment device may be formed as a long bag-like device and may be used with minimally invasive techniques.

The osteoinductive or biologically active bone matrix composition may be combined with a carrier such as a polymer carrier and molded into a solid implant. U.S. Patent Nos. 6,696,073, 6,478,825, 6,440,444, and 6,294,187 and U.S. Patent Publications Nos. 2006/0216323 and 2005/0251267 disclose such implants and methods for making such implants.

### X. Formulation of Bone Matrix Containing Compositions

The osteoinductive or biologically active bone matrix composition, the carrier, or the osteoimplant may be formulated for a particular use. The formulation may be used to alter the physical, biological, or chemical properties of the carrier. A physician would readily be able to determine the formulation needed for a particular application, taking into account such factors as the type of injury, the site of injury, the patient's health, and the risk of infection. In various embodiments, the composition may comprise, for example less than approximately 0.5% water, less than approximately 1% water, or less than approximately 5% water.

Osteoinductive or biologically active bone matrix compositions, carriers, or osteoimplants therefore may be prepared to have selected resorption/loss of osteoinductivity rates, or even to have different rates in different portions of an implant. For example, the formulation process may include the selection of partially demineralized particles of a particular size or composition, combined with the selection of a particular stabilizing agent or agents, and the amounts of such agents.

Physical properties such as deformability and viscosity of the carrier may also be chosen depending on the particular clinical application. The bone matrix provided herein may be mixed with partially demineralized bone and/or other materials and factors to improve other characteristics of the implant. For example, the bone matrix may be mixed with other agents to improve wound healing. These agents may include drugs, proteins, peptides, polynucleotides, solvents, chemical compounds, and biological molecules.

Further, the bone matrix composition may be formulated to be settable and/or injectable. Thus, for example, the composition may be injectable through a 10-gauge, a 12-gauge, or an 18-gauge needle.

Accordingly, the bone matrix composition may be rubbery, rubbery with chunks, stiff (as freeze-dried), stiff with chunks, putty, paste, flowable, or injectable. The term "flowable" in this context applies to compositions whose consistencies range from those which can be described as shape-sustaining but readily deformable, e.g., those which behave like putty, to those which are runny. Specific forms of flowable bone powder compositions include cakes, pastes, creams and fillers. Reference is made to U.S. Patent No. 5,290,558 for discussion of flowable materials.

Also, as previously discussed, the osteoinductive or biologically active bone matrix composition may be formed into various shapes and configurations, including rods, strings, sheets, weaves, solids, cones, discs, fibers, and wedges. Such shapes may result from a monolithic bone piece or an aggregate of bone particles. In certain embodiments, the shape and size of the bone matrix affect the time course of osteoinductivity. For example, in a cone or wedge shape, the tapered end will result in osteoinductivity shortly after implantation of the osteoimplant, whereas the thicker end will lead to osteoinductivity later in the healing process (hours to days to weeks later). In certain embodiments, bone matrix osteoimplants may include an aggregate of bone particles, the particles have a length of greater than 2 mm, greater than 1.5 mm, greater than 1 mm, greater than 500 microns, or greater than 200 microns across its widest dimension. Also, larger particle size will induce bone formation over a longer time course than smaller particles. Particles of different characteristics (e.g., composition, size, shape) may be used in the formation of these different shapes and configurations. For example, the osteoimplant may include a sheet of partially demineralized bone, with a layer of long half-life particles alternated between layers of shorter half-life particles. See U.S. Patent No. 5,899,939for suitable examples. In a weave, strands composed of short half-life particles may be woven together with strands of longer half-lives.

### XI. Conclusion

Bone matrix compositions and, more specifically, bone matrix compositions having nanofibrous structures are disclosed and methods for their production are provided. The bone matrix compositions may comprise demineralized bone, partially demineralized bone or surface demineralized bone. A DBM composition may be provided wherein greater than about 1%, about 10%, about 20%, about 30%, about 40%, about 50%, about 60%, about 70%, about 80%, about 90%, or about 99% of the DBM particles have nanofibrous structures. Mineralized or surface demineralized bone may be provided wherein greater than about 1%, about 10%, about 20%, about 30%, about 40%, about 50%, about 60%, about 70%, about 80%, about 90%, or about 99% of the particles have nanofibrous structures. Bone matrix having a predetermined moisture content, for example, about 15%, about 8%, about 6%, about 3%, or about 1%, and having greater than about 40%, about 50%, about 60%, about 70%, about 75%, about 80%, about 90%, or about 99% non-denatured collagen are provided. Compositions may be provided comprising bone matrix and extract wherein the particle surfaces are at least greater than approximately 20 % nanofibrous in nature. The nanofibrous structures may be provided by processing the bone such that the native nanofibrous structure of the bone is maintainedThe nanofibrous structure may be imparted to the bone by adding nanofibrous materials to the bone.

## Claims

1. A method for producing a demineralized bone matrix composition, the method comprising:
providing bone;
demineralizing the bone;
drying the bone such that, after drying, the bone matrix has a residual moisture content of 15% or less;
and
imparting nanostructures to the bone to provide a nanoscale textured surface; wherein the imparting step is selected from
adsorption,
coating,
precipitating,
treating biomacromolecules of the bone to form surfaces with charged polycationic or anionic polyelectrolytes or with different hydrophilic and hydrophobic domains so that biomacromolecules with opposite charges or hydrophilic / hydrophobic properties are immobilized on the bone surface, and
electrospinning.

2. The method of Claim 1, wherein the imparting step is electrospinning.

3. The method of Claim 2, wherein the nanostructures are synthetic polymers selected from poly(lactide) (PLLA), poly(lactide-co-glycolide) (PLGA), polycaprolactone (PCL), and poly(L-lysine).

4. The method of Claim 2, wherein the nanostructures are naturally derived polymers selected from collagen, gelatin, chitosan, hyaluronic acid, and alginate.

5. The method of Claim 1, wherein the nanostructures contain encapsulated biological active molecules selected from antibiotics, functional peptides, proteins, growth factors, cytokines, DNAs, RNAs, siRNAs and any combination each thereof.

6. The method of any one of Claims 1-5, further comprising adding extract to the bone.

7. The method of any one of Claims 1-6, wherein the nanostructures are selected from nanofibres, nanoparticles, nanospheres, and nanomicelles.

8. The method of any one of Claims 1-7, wherein the nanostructures range from 40 nm to 600 nm in at least one dimension.

9. A demineralized bone matrix composition produced according to the method of any one of Claims 1-8.

## Patentansprüche

1. Verfahren zur Herstellung einer demineralisierten Knochenmatrixzusammensetzung, das Verfahren Folgendes umfassend: Bereitstellen von Knochen;
Demineralisierung des Knochens;
Trocknen des Knochens, so dass die Knochenmatrix nach dem Trocknen einen Restfeuchtigkeitsgehalt von 15 % oder weniger aufweist; und
Verleihen von Nanostrukturen an den Knochen, um eine nanoskalige strukturierte Oberfläche bereitzustellen, wobei der verleihende Schritt ausgewählt ist aus Adsorption, Beschichtung, Fällung, Behandlung von Biomakromolekülen des Knochens, um Oberflächen mit geladenen polykationischen oder anionischen Polyelektrolyten oder mit verschiedenen hydrophilen und hydrophoben Domänen zu bilden, so dass Biomakromoleküle mit entgegengesetzten Ladungen oder hydrophilen/hydrophoben Eigenschaften auf der Knochenoberfläche immobilisiert werden, und Elektrospinnen.

2. Verfahren nach Anspruch 1, wobei der verleihende Schritt das Elektrospinnen ist.

3. Verfahren nach Anspruch 2, wobei die Nanostrukturen synthetische Polymere sind, ausgewählt aus Poly(Lactid) (PLLA), Poly(Lactid-Co-Glykolid) (PLGA), Polycaprolacton (PCL) und Poly(L-lysin).

4. Verfahren nach Anspruch 2, wobei die Nanostrukturen natürlich abgeleitete Polymere sind, ausgewählt aus Kollagen, Gelatine, Chitosan, Hyaluronsäure und Alginat.

5. Verfahren nach Anspruch 1, wobei die Nanostrukturen eingekapselte biologisch aktive Moleküle enthalten, ausgewählt aus Antibiotika, funktionellen Peptiden, Proteinen, Wachstumsfaktoren, Zytokinen, DNAs, RNAs, siRNAs und jeder beliebigen Kombination davon.

6. Verfahren nach einem der Ansprüche 1 bis 5, ferner umfassend die Zugabe von Extrakt zum Knochen.

7. Verfahren nach einem der Ansprüche 1 bis 6, wobei die Nanostrukturen aus Nanofasern, Nanopartikeln, Nanosphären und Nanomizellen ausgewählt sind.

8. Verfahren nach einem der Ansprüche 1 bis 7, wobei die Nanostrukturen von 40 nm bis 600 nm in mindestens einer Dimension reichen.

9. Demineralisierte Knochenmatrixzusammensetzung, hergestellt nach dem Verfahren nach einem der Ansprüche 1 bis 8.

## Revendications

1. Procédé de production d'une composition de matrice osseuse déminéralisée, le procédé comprenant les étapes consistant à :fournir un os ;
déminéraliser l'os ;
sécher l'os de façon qu'après séchage, la matrice osseuse ait une humidité résiduelle inférieure ou égale à 15 % ; et
transférer des nanostructures dans l'os pour fournir une surface texturée à l'échelle nanométrique ; l'étape de transfert étant choisie entre l'adsorption, le revêtement, la précipitation, le traitement de biomacromolécules de l'os pour former des surfaces avec des polyélectrolytes chargés cationiques ou anioniques ou avec différents domaines hydrophiles et hydrophobes, de sorte que des biomacromolécules avec des charges opposées ou des propriétés hydrophiles / hydrophobes opposées soient immobilisées à la surface de l'os, et l'électrofilage.

2. Procédé selon la revendication 1, dans lequel l'étape de transfert est un électrofilage.

3. Procédé selon la revendication 2, dans lequel les nanostructures sont des polymères synthétiques choisis parmi le poly(lactide) (PLLA), le poly(lactide-co-glycolide) (PLGA), la polycaprolactone (PCL) et la poly(L-lysine).

4. Procédé selon la revendication 2, dans lequel les nanostructures sont des polymères d'extraction naturelle choisis parmi le collagène, la gélatine, le chitosane, l'acide hyaluronique et l'alginate.

5. Procédé selon la revendication 1, dans lequel les nanostructures contiennent des molécules biologiques actives encapsulées choisies parmi des antibiotiques, des peptides fonctionnels, des protéines, des facteurs de croissance, des cytokines, des ADN, des ARN, des pARNi et toute combinaison de ceux-ci.

6. Procédé selon l'une quelconque des revendications 1 à 5, comprenant en outre l'ajout d'un extrait à l'os.

7. Procédé selon l'une quelconque des revendications 1 à 6, dans lequel les nanostructures sont choisies parmi des nanofibres, des nanoparticules, des nanosphères et des nanomicelles.

8. Procédé selon l'une quelconque des revendications 1 à 7, dans lequel les nanostructures ont au moins une dimension comprise entre 40 nm et 600 nm.

9. Composition de matrice osseuse déminéralisée selon le procédé selon l'une quelconque des revendications 1 à 8.
